# EUROPÄISCHE PATENTSCHRIFT

(11) **EP 1 052 936 B1**
(45) Veröffentlichungstag und Bekanntmachung des Hinweises auf die Patenterteilung: **23.01.2008**
(21) Anmeldenummer: 99961019.9
(22) Anmeldetag: 26.11.1999
(51) Int. Cl.: A61B 6/00

(54) **RÖNTGENUNTERSUCHUNGSEINRICHTUNG UND VERFAHREN ZUR ERZEUGUNG VON VERZERRUNGSFREIEN RÖNTGENBILDERN**
X-RAY EXAMINATION DEVICE AND METHOD FOR PRODUCING UNDISTORTED X-RAY IMAGES
SYSTEME DE RADIOGRAPHIE PAR RAYONS X ET PROCEDE POUR PRODUIRE DES IMAGES DE RAYONS X SANS DISTORSION

(30) Priorität: 08.12.1998 DE 19856537
(43) Veröffentlichungstag der Anmeldung: 22.11.2000
(73) Patentinhaber: Koninklijke Philips Electronics N.V., 5621 BA Eindhoven (NL); Philips Intellectual Property & Standards GmbH, 20099 Hamburg (DE)
(72) Erfinder: ZYLKA, Waldemar, NL-5656 AA Eindhoven (NL); SABCZYNSKI, Jorg, NL-5656 AA Eindhoven (NL); WEESE, Jürgen, NL-5656 AA Eindhoven (NL)
(74) Vertreter: Kopf, Korbinian Paul
(86) Internationale Anmeldenummer: PCT/EP1999/009225
(87) Internationale Veröffentlichungsnummer: WO 2000/033739

(56) Entgegenhaltungen:
- WO-A-97/48339
- DE-A- 19 703 556
- FR-A- 2 631 810
- US-A- 5 748 768
- US-A- 5 772 594

## Beschreibung

Die Erfindung betrifft ein Röntgenuntersuchungsgerät und ein Verfahren zur Erzeugung von verzerrungsfreien Röntgenbildern.

Bewegliche Röntgenuntersuchungsgeräte, beispielsweise C-Bogen Röntgengeräte, werden bei bestimmten Operationen, z.B. in der Orthopädie benutzt. Die Besonderheit, die eine regelmäßige Kontrolle der Röntgenbilder auf Verzerrungen erfordert, wird durch die beweglichen Baugruppen eines solchen Röntgenuntersuchungsgerätes und die ständig veränderten Aufnahmebedingungen hervorgerufen. Auch in der Computer unterstützten Chirurgie (computer aided surgery) finden deratige Verfahren und Vorrichtungen ihre Anwendung.

In der US 5 772 594 wird ein C-Bogen-Röntgensystem beschrieben, bei dem am Bildverstärker mehrere Referenzsensoren angeordnet sind, die von einem Positionserfassungssystem geortet werden. Am zu behandelnden Knochen sind Marker angeordnet, die von diesem Positionserfassungssystem geortet werden. Bei dieser Anordnung wird das einmal aufgenommene Bild mit einer Bildverarbeitungsanordnung angezeigt und die Operationswerkzeuge des Chirurgen, die ebenfalls mit Sensoren versehen sind, werden von dem Positionserfassungssystem erfaßt und über das angezeigte Röntgenbild des abgebildeten Knochens eingeblendet. Veränderungen, die während der Operation nach Aufnahme des Bildes an der Lage des Knochens auftreten, werden nicht abgebildet.

Geräte und Verfahren gemäß der Oberbegriffe der Ansprüche 1 bzw. 7 sind aus US 5 748 768, WO 97/48339 und FR 2 631 810 bekannt.

Bilder, die von C-Bogen Röntgenuntersuchungsgeräten geliefert werden, sind in der Regel verzerrt. Diese Verzerrungen entstehen einerseits durch die gekrümmte Oberfläche des Bildverstärkers und andererseits durch Veränderungen des äußeren Magnetfeldes, z.B. dem Erdmagnetfeld. Außerdem wird beispielsweise der C-Bogen durch das Gewicht des Bildverstärkers und des Röntgenstrahlers Verbiegungen ausgesetzt, die nicht konstant sind. Derartige Verbiegungen und Verzerrungen führen zu Änderungen der Abbildungseigenschaften und damit zu fehlerhaften Röntgenaufnahmen. Mobile C-Bögen können vom Chirurgen jederzeit an eine andere Stelle oder in eine andere Ausrichtung bewegt werden, wodurch die Abbildungseigenschaften ständig verändert werden.

Für neuartige Operationstechniken, wie CAS (Computer Aided Surgerey), ist es notwendig die vollständigen Abbildungseigenschaften für die C-Bogen Röntgenanordnung zu kennen.

Auch für Anwendungen im Bereich der Tomosynthese und der Computertomographie ist es notwendig für die Rekonstruktion der Röntgenbilder die Abbildungseigenschaften der jeweiligen Röntgengeräte genau zu kennen.

Aufgabe der Erfindung ist es deshalb, ein Verfahren und eine Vorrichtung anzugeben, mit denen eine Korrektur der Verzerrungen in den Patientenaufnahmen möglich ist.

Erfindungsgemäß wird diese Aufgabe dadurch gelöst, daß das Röntgenuntersuchungsgerät wenigstens einen Kalibrationskörper zur Erzeugung eines Referenzmusters und eine Korrektureinheit gemäß Anspruch 1 aufweist.

Die Positionen der Gehäuse des Bildverstärkers und des Röntgenstrahlers werden mit Hilfe einer Positionserfassungsanordnung bezüglich eines festen Raum-Koordinatensystem bestimmt. Dazu sind an den Gehäusen des Bildverstärkers und des Röntgenstrahlers Referenzsensoren angebracht, die je ein Bildverstärker- und ein Röntgenstrahlerkoordinatensystem, das ortsfest zum jeweiligen Gehäuse ist, definieren. Außerdem sind am Bildverstärker und am Röntgenstrahler oder auch an beiden geeignete Kalibrationskörper angebracht. Die Position dieser Kalibrationskörper ist ortsfest zum jeweiligen Gehäuse des Bildverstärkers oder des Röntgenstrahlers und somit kann bezüglich des jeweiligen Koordinatensystems das den Kalibrationskörpern zugeornete Referenzmuster bestimmt werden. Dazu wird die Position der Kalibrationkörper in dem von dem Positionerfassungsgerät gebildeten Koordinatensystem berechnet.

Die Kalibrationskörper haben eine feste und bekannte Form und Gestalt. Sie werden in der Patientenröntgenaufnahme als tatsächliche Muster zusammen mit dem Patienten abgebildet und identifiziert. Aus der bekannten Form und Gestalt, wie auch aus der bekannten Position der Kalibrationskörper zu den Gehäusen des Bildverstärkers und des Röntgenstrahlers im berechneten Referenzmuster und der Position der Kalibrationskörper in den Patientenröntgenaufnahmen, lassen sich die Abbildungseigenschaften des Röntgenuntersuchungsgeräts bestimmen. Mit der Bestimmung der Abbildungseigenschaften ist es möglich, Verzerrungen, die beispielsweise durch die gekrümmte Oberfläche des Bildverstärkers, durch eine Änderung des Erdmagnetfeldes oder durch Verbiegungen des C-Bogens entstehen, zu korrigieren.

Als vorteilhaft erweist es sich auch, die Position der Kalibrationskörper mit der Positionserfassungsanordnung direkt zu erfassen. Die Position wird mit geeigneten Zeigemitteln, die mit Referenzsensoren versehen sind, erfaßt und mittels der Referenzsensoren zu der Positionserfassungsanordnung übertragen. Aus den bekannten Positionsdaten der Kalibrationskörper und den Positionen der insbesondere am Rand abgebildeten Kalibrationskörper in den Patientenröntgenaufnahmen werden dann die Abbildungseigenschaften des Röntgengeräts berechnet.

Dadurch ist es möglich, eine von Verzerrungen bereinigte Patientenröntgenaufnahme mit einer einzigen Patientenröntgenaufnahme ohne mehrere Kalibrationsaufnahmen oder mehreren Patientenröntgenaufnahmen zu erzeugen. Die Korrektur der Verzerrung erfolgt intraoperativ, so daß dem Betrachter ein verzerrungsfreies Röntgenbild dargestellt wird.

Bei einer Ausgestaltung der Erfindung erweist es sich als vorteilhaft, daß die Kalibrationskörper in einer für Röntgenstrahlen durchlässigen Scheibe z.B. kreisförmig angeordnet sind und aus Röntgenstrahlen absorbierenden Kugeln oder Fäden bestehen. Die für Röntgenstrahlung durchsichtige Scheibe, beispielsweise aus Plexiglas, absorbiert die Röntgenstrahlen nicht, so daß die Patientenaufnahme dadurch nicht zusätzlich verfälscht wird. Die Kugeln oder Fäden hingegen lassen sich durch ihre hohe Absorption sehr gut in den Patientenaufnahmen erkennen.

Diese Plexiglasscheiben mit den kreisförmig angeordneten Metallkugeln oder den gekreuzten Metallfäden werden vor dem Bildverstärker und/oder dem Röntgenstrahler in einem jeweils definierten Abstand angebracht. Aus den Verschiebungen oder Differenzen zwischen den Positionen der Kalibrationskörper in dem errechneten Referenzmuster und dem tatsächlichen erzeugtem Muster der Kalibrationskörper in der Patientenröntgenaufnahme ist es möglich, die Abbildungseigenschaften des Röntgenuntersuchungsgerätes festzustellen und auftretende Verzerrungen in der Patientenröntgenaufnahme zu korrigieren.

Bei der Computertomographie (CT) wird aus mehreren Schnittbildern ein dreidimesionales Röntgenbild rekonstruiert. Da auch hier äußerer Magnetfelder auf das CTgerät wirken und Verschiebungen zwischen Röntgenstrahler und Detektor die Patientenröntgenaufnahme negativ beeinflussen, ist für die Rekonstruktion eines verzerrungsfreien Röntgenbildes die Kenntnis der Abbildungseigenschaften des CT-gerätes notwendig. Die Kalibrationskörper werden in diesem Fall so angeordnet, daß sie in jedem Schnittbild sichtbar sind und mittels des berechneten Referenzmusters der Kalibrationskörper und den tatsächlich erzeugten Mustern der Kalibrationskörper in der Patientenröntgenaufnahme die Abbildungseigenschaften des CT-geräts ermittelt werden können.

Für die Tomosynthese, bei der ebenfalls Schnittbilder aufgenommen werden, ist durch den Einsatz von Kalibrationskörpern und der Feststellung der Abbildungseigenschaften die erfidungsgemäße Korrektur von Verzerrungen möglich.

Nachfolgend wird ein Ausführungsbeispiel anhand der Zeichnungen näher erläutert.
Fig. 1 zeigt eine schematische Darstellung einer C-Bogen Röntgenanordnung.
Fig. 2 Kalibrationskörper des Bildverstärkers
Fig. 3 Kalibrationskörper am Röntgenstrahler
Fig. 4 verzerrte Patientenröntgenaufnahme
Fig. 5 entzerrte Patientenröntgenaufnahme

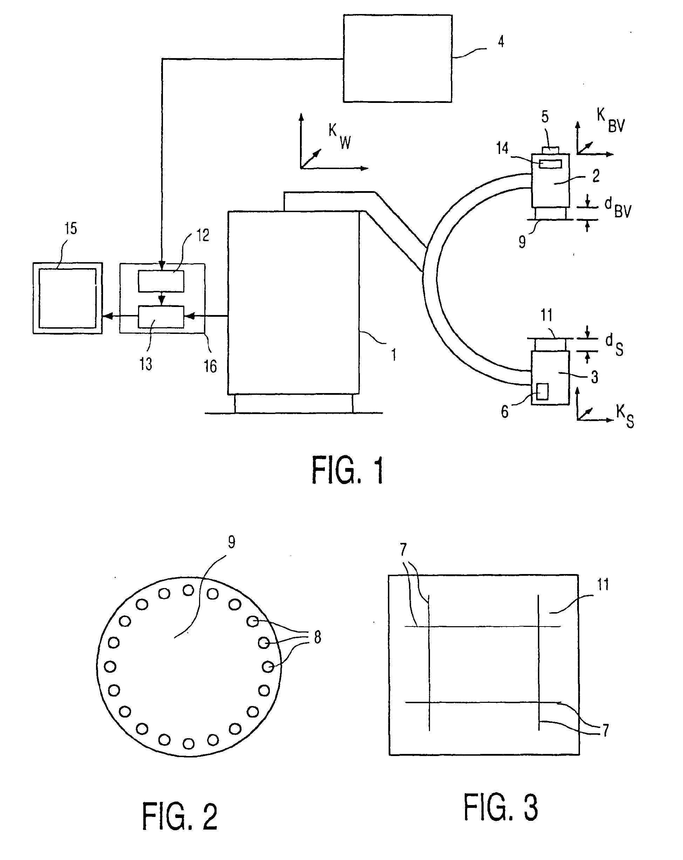

In Fig. 1 weist die C-Bogen Röntgenanordnung ein Gehäuse 1 zur Befestigung des C-Bogens, an dem ein Bildverstärker 2 und ein Röntgenstrahler 3 angeordnet sind, auf. Am Bildverstärker 2 sind Referenzsensoren 5 angeordnet, die bei einer beispielsweise optischen Positionserfassungsanordnung 4 als LED's realisiert sein können. Diese Referenzsensoren 5 übertragen die Koordinaten des Bildverstärkergehäuses 2 zur Positionserfassungsanordnung 4. Am Röntgenstrahler 3 sind ebenfalls Referenzsensoren 6 angeordnet, die der Positionserfassungsanordnung 4 die Koordinaten des Röntgenstrahlers 3 übermitteln. Mit Hilfe dieser Referenzsensoren 5 und 6 wird jeweils ein Koordinatensystem, das ortsfest zum jeweiligen Gehäuse ist (Bildverstäkerkoordinatensystem K_{BV} bzw. Röntgenstrahlerkoordinatensystem K_{S}) definiert. Mit der Positionserfassungsanordnung 4 wird ein Raum-Koordinatensystem K_{W} gebildet, in dem die Lage der Koordinatensysteme K_{BV} und K_{S} durch das Übertragen der Positionen mittels der Referenzsensoren 5 und 6 bekannt ist. Vor der Öffnung des Bildverstärkers 2 für den Eintritt der vom Röntgenstrahler 3 ausgesandten Röntgenstrahlen sind in einem definierten Abstand d_{BV} Kalibrationskörper 8 angebracht.

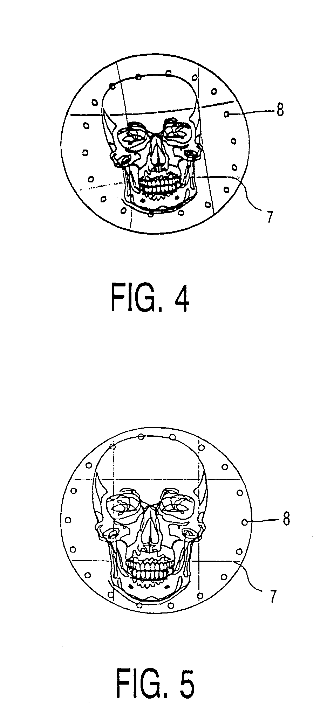

Die Kalibrationskörper 8 sind in Figur 2 dargestellt und sind beispielsweise kleine Metallkugeln, die kreisförmig am Rand einer durchsichtigen Plexiglasscheibe 9 angeordnet sind. An dem Röntgenstrahler 3 sind in einem definiertem Abstand dₛ auf einer ebenfalls durchsichtigen Plexiglasscheibe 11 gekreuzte Metalldrähte eingelassen, die als in Figur 3 dargestellten Kalibrationskörper 7 einen Röntgenschatten ergeben. Mit Hilfe der bekannten Abstände dₛ und d_{BV} und der bekannten Geometrie der Kalibrationskörper 7 und 8 in den jeweiligen Plexiglasscheiben vor dem Bildverstärker 2 und vor dem Röntgenstrahler 3 läßt sich die genaue Position der Kalibrationskörper in bezug auf den Bildverstärker 2 oder den Röntgenstrahler 3 und demzufolge auch für das jeweilige Koordinatensystem K_{BV} und Ks ermitteln. Der Röntgenstrahler 3 und der Bildverstärker 2 sind mit den ihrerseits zugeordneten Koordinatensystemen K_{BV} und K_{S} von der Positionserfassungsanordnung 4 eindeutig in dem Raum-Koordinatensystem K_{W} lokalisiert. Die Positionen der Kalibrationskörper 7 und 8 am Bildverstärker 2 und Röntgenstrahler 3 werden von der Positionserfassungsanordnung 4 an eine in der Bildverarbeitungseinheit 16 befindliche Berechnungseinheit 12 übertragen. Dort wird das Referenzmuster berechnet. Im Bildverstärker 2 ist eine CCD-Kamera 14 angeordnet, die die in sichtbare Strahlung umgewandelte Röntgenstrahlung aufnimmt. Von dort wird die Patientenröntgenaufnahme mit den auf ihr abgebildeten Kalibrationskörpern 7 und 8 an die Berechungseinheit 12 geleitet. Anhand der Positionen der Kalibrationskörper 7 und 8 im Referenzmuster und der Position der Kalibrationskörper 7 und 8 in der Patientenröntgenaufnahme lassen sich die Abbildungseigenschaften der C-Bogen Röntgenanordnung bestimmen und durch einen Vergleich der Positionen Unterschiede in diesen feststellen. Unterschiede in den Positionen zeigen Verzerrungen an, die dann in der Korrektureinheit 13 unter Berücksichtigung der errechneten Abbildungseigenschaften entzerrt werden. Figur 4 zeigt eine verzerrte Patientenröntgenaufnahme. Auf der Anzeigeeinheit 15 wird das entzerrte Bild( Fig.5) des Patienten dargestellt.

Mit Hilfe eines Zeigeinstrumentes, welches hier nicht dargestellt ist, aber von der Positionserfassungsanordnung 4 durch angebrachte Referenzsensoren geortet wird, ist es möglich, die Position der Kalibrationskörper direkt zu erfassen.

In einer weiteren Ausführungsform wird nur der Bildverstärker 2 mit Hilfe der Positionserfassungsanordnung 4 vermessen. Die Position des Bildverstärker-Koordinatensystems K_{BV} ist damit relativ zum äußeren Koordinatensystem K_{W} bekannt. Aus der Position der Kalibrationskörper 7 des Röntgenstrahlers 3 in der Patientenaufnahme kann dann zusammen mit Kenntnis der Form und Position dieser Kalibrationskörper 7 im Strahlerkoordinatensystem K_{S} die Position des Fokuspunktes des Röntgenstrahlers 3 im Bildverstärker-Koordinatensystem K_{BV} oder im äußeren Koordinatensystem K_{W} berechnet werden. Damit lassen sich Verzerrungen, die aus Verbiegungen der C-Bogen Röntgenanordnung resultieren, in der Patientenröntgenaufnahme korrigieren.

In einer dritten Ausführungsform wird nur der Röntgenstrahler 3 mit Hilfe der Positionserfassungsanordnung 4 vermessen. Die Position des Strahlerkoordinatensystems K_{S} ist damit relativ zum äußeren Koordinatensystem K_{W} bekannt. Aus der Position der Kalibrationskörper 8 des Bildverstärkers 2 in der Patientenröntgenaufnahme kann dann zusammen mit Kenntnis der Form und Position dieser Kalibrationskörper 8 im Bildverstärker-Koordinatensystem K_{BV} die Position des Bildverstärkers 2 im Röntgenstrahlerkoordinatensystem K_{S} oder im äußeren Koordinatensystem K_{W} berechnet werden. Auch damit lassen sich Verzerrungen in der Patientenröntgenaufnahme korrigieren.

Als vorteilhaft erweist es sich, den Röntgenstrahler 3 und auch den Bildverstärker 2 mit Kalibrationskörpern 7 und 8 auszurüsten und beide mit Referenzsensoren 5 und 6 auszustatten, so daß die Möglichkeit besteht, beide vermessen zu können. Wenn die Position des Röntgenstrahlers 3 und auch des Bildverstärkers 2 nicht bestimmt werden kann, dann wird jeweils nur das sichtbare andere mit Referenzsensoren versehene Gehäuse des Röntgenstrahlers 3 oder des Bildverstärkers 2 zur Bestimmung der Abbildungseigenschaften des C-Bogens benutzt. Dieser Fall tritt bei optischen Positionserfassungsanordnungen häufig ein.

Bei einer weiteren Ausgestaltung der Erfindung ist es vorgesehen, mit Methoden der Bildverarbeitung die Kalibrationskörper 7 und 8, die in den Patientenaufnahmen auch abgebildet sind, durch Verkleinerung des Bildfeldes aus den Patientenröntgenaufnahmen zu entfernen. Damit steht dem Chirurgen ein unverfälschtes Bild des Patienten zur Verfügung.

Die Referenzsensoren können auch durch rein geometrisch unterscheidbare Formen realisiert sein. In diesem Fall erkennt die Positionserfassungsanordnung diese Formen und erfaßt deren Position.

Die Kalibrationskörper können zusätzlich oder auch ausschließlich am Patienten oder am Operationstisch angebracht sein, so daß diese in der Patientenröntgenaufnahme abgebildet werden. Durch an den Kalibrationskörpern angebrachten Referenzsensoren ist deren Position für die Berechnung der Abbildungseigenschaften bekannt.

## Patentansprüche

1. Röntgenuntersuchungsgerät (1) zur Erzeugung von verzerrungsfreien Röntgenbildern mit wenigstens einem Kalibrationskörper (7,8) zur Erzeugung eines Referenzmusters und einer Korrektureinheit (13), **dadurch gekennzeichnet, daß** die Korrektureinheit zur Korrektur von Verzerrungen in einer Patientenröntgenaufnahme mittels des tatsächlich erzeugten Musters der Kalibrationskörper (7,8) in der Patientenröntgenaufnahme und des berechneten Referenzmusters eingerichtet ist.

2. Röntgenuntersuchungsgerät nach Anspruch 1, **dadurch gekennzeichnet, daß** eine Positionserfassungsvorrichtung (4) zur Erfassung der Positionen von an einem Röntgenstrahler (3) und/oder an einem Bildverstärker (2) angeordneten Kalibrationskörpern (7,8) mittels am Röntgenstrahler (3) und/oder am Bildverstärker (2) angeordneten Referenzsensoren (5,6) und eine Berechnungseinheit (12) zur Berechnung der Referenzmuster aus den bekannten Abmessungen des Röntgenuntersuchungsgerätes (1) und den erfaßten Positionen der Kalibrationskörper (7,8) und eine Anzeigeeinheit (15) zur Darstellung der verzerrungsfreien Röntgenbilder vorgesehen sind.

3. Röntgenuntersuchungsgerät nach den Ansprüchen 1 und 2, **dadurch gekennzeichnet, daß** die Position eines Kalibrationskörpers (7,8) mittels eines mit Referenzsensoren versehenen Zeigegerätes erfaßbar ist.

4. Röntgenuntersuchungsgerät nach den Ansprüchen 1 bis 3, **dadurch gekennzeichnet, daß** wenigstens ein Kalibrationskörper (7,8) in einer im Röntgenstrahlengang angeordneten, für Röntgenstrahlung durchsichtigen Scheibe (9,11) angeordnet ist.

5. Röntgenuntersuchungsgerät nach den Ansprüchen 1 bis 4, **dadurch gekennzeichnet, daß** Kalibrationskörper (7,8) aus Röntgenstrahlen absorbierenden Körpern gebildet sind.

6. Röntgenuntersuchungsgerät nach den Ansprüchen 1 bis 5, **dadurch gekennzeichnet, daß** weitere Kalibrationskörper im Röntgenstrahlengang dem Patienten zugeordnet sind oder am Operationstisch angeordnet sind.

7. Verfahren zur Erzeugung von verzerrungsfreien Röntgenbildern, **dadurch gekennzeichnet, daß** ein Kalibrationskörpern (7,8) zugeordnetes Referenzmuster berechnet wird, eine Patientenröntgenaufnahme mit Kalibrationskörpern (7,8) aufgenommen wird und eine Korrektur von Verzerrungen in der Patientenröntgenaufnahme mittels des in der Patientenröntgenaufnahme tatsächlich erzeugten Musters der Kalibrationskörper (7,8) und des berechneten Referenzmusters vorgenommen wird.

## Claims

1. An X-ray examination apparatus (1) for the generation of distortion-free images, which apparatus (1) comprises at least one calibration member (7, 8) for generating a reference pattern and a correction unit (13), **characterized in that** said correction unit is designed for correcting distortions in an X-ray image of a patient on the basis of the pattern of the calibration members (7, 8) actually formed in the X-ray image of the patient and on the basis of the calculated reference pattern.

2. An X-ray examination apparatus as claimed in Claim 1, **characterized in that** there are provided a position-measuring device (4) for measuring the positions of calibration members (7, 8), arranged on an X-ray source (3) and/or on an image intensifier (2) by means of reference sensors (5, 6) arranged on the X-ray source (3) and/or on the image intensifier (2), an arithmetic unit (12) for calculating the reference pattern from the known dimensions of the X-ray examination apparatus (1) and the measured positions of the calibration members (7, 8), and a display unit (15) for displaying the distortion-free X-ray images.

3. An X-ray examination apparatus as claimed in the Claims 1 and 2, **characterized in that** the position of a calibration member (7, 8) can be detected by means of an indicator device provided with reference sensors.

4. An X-ray examination apparatus as claimed in the Claims 1 to 3, **characterized in that** at least one calibration member (7, 8) is provided in a disc (9, 11) which is arranged in the X-ray beam path and is transparent to X-rays.

5. An X-ray examination apparatus as claimed in the Claims 1 to 4, **characterized in that** the calibration members (7, 8) are constructed as X-ray absorbing members.

6. An X-ray examination apparatus as claimed in the Claims 1 to 5, **characterized in that** further calibration members in the X-ray beam path are associated with the patient or are mounted on the operating table.

7. A method of forming distortion-free X-ray images, **characterized in that** a reference pattern associated with calibration members (7, 8) is calculated, an X-ray image of a patient is formed in which calibration members (7, 8) are reproduced, and distortions in the X-ray image of the patient are corrected on the basis of the pattern of calibration members (7, 8) actually formed in the X-ray image of the patient and on the basis of the calculated reference pattern.

## Revendications

1. Appareil d'examen radiographique (1) en vue de la production d'images radiographiques sans distorsion doté d'au moins un corps de calibrage (7, 8) pour la production d'un modèle de référence et d'une unité de correction (13), **caractérisé en ce que** l'unité de correction est conçue pour la correction de distorsions dans un cliché radiographique d'un patient à l'aide du modèle effectivement produit des corps de calibrage (7, 8) dans le cliché radiographique du patient et du modèle de référence calculé.

2. Appareil d'examen radiographique selon la revendication 1, **caractérisé en ce qu'**un dispositif capteur de position (4) est prévu pour l'enregistrement des positions de corps de calibrage (7, 8) disposés sur un générateur de rayons X (3) et/ou sur un amplificateur d'images (2) à l'aide de capteurs de référence (5, 6) disposés sur le générateur de rayons X (3) et/ou sur l'amplificateur d'images (2) et une unité de calcul (12) pour le calcul du modèle de référence à partir des dimensions connues de l'appareil d'examen radiographique (1) et des positions enregistrées des corps de calibrage (7, 8) et une unité d'affichage (15) pour la représentation des images radiographiques sans distorsion.

3. Appareil d'examen radiographique selon l'une des revendications 1 et 2, **caractérisé en ce que** la position d'un corps de calibrage (7, 8) peut être enregistrée à l'aide d'un appareil d'affichage doté de capteurs de référence.

4. Appareil d'examen radiographique selon l'une des revendications 1 à 3, **caractérisé en ce qu'**au moins un corps de calibrage (7, 8) est disposé dans un disque (9, 11) transparent aux rayons X et disposé sur le trajet des rayons X.

5. Appareil d'examen radiographique selon l'une des revendications 1 à 4, **caractérisé en ce que** les corps de calibrage (7, 8) sont formés à partir de corps absorbant des rayons X.

6. Appareil d'examen radiographique selon l'une des revendications 1 à 5, **caractérisé en ce que** d'autres corps de calibrage sont attribués au patient sur le trajet des rayons X ou sont disposés sur la table d'opération.

7. Procédé de production d'images radiographiques sans distorsion, **caractérisé en ce qu'**un modèle de référence attribué à des corps de calibrage (7, 8) est calculé, un cliché radiographique de patient est enregistré avec des corps de calibrage (7, 8) et une correction des distorsions dans le cliché radiographique du patient est effectuée à l'aide du modèle effectivement produit des corps de calibrage (7, 8) et du modèle de référence calculé dans le cliché radiographique du patient.
